# EUROPEAN PATENT APPLICATION

(11) **EP 1 358 869 A1**
(43) Date of publication of application: **05.11.2003**
(21) Application number: 02009588.1
(22) Date of filing: 26.04.2002
(51) Int. Cl.: A61K 7/46, A61K 7/48, A61K 38/08

(54) **Biologically active compounds for the modificaton of bodily odours**

(71) Applicant: Max-Planck-Gesellschaft zur Förderung der Wissenschaften e.V. Berlin, 80539 München (DE)
(72) Inventor: Boehm, Prof. Dr. Thomas, 79279 Vörstetten (DE); Milinski, Prof. Dr. Manfred, 24306 Plön (DE)
(74) Representative: Weiss, Wolfgang, Dipl.-Chem. Dr.

(57) **Abstract**

The invention relates to the use of MHC ligands, e.g. MHC-binding peptides, for the manufacture of an agent for modifying bodily odours. Particularly, the invention is applicable in the field of cosmetics and medicine.

## Description

The invention relates to the use of MHC ligands, e.g. MHC-binding peptides, for the manufacture of an agent for modifying bodily odours. Particularly, the invention is applicable in the field of cosmetics and medicine.

Major histocompatibility complex (MHC) molecules present self and nonself peptides at the cell surface for recognition by T-lymphocytes. In natural populations, MHC genes are highly polymorphic(1); variability among MHC molecules leads to markedly different ligand specificities(2) and thereby controls resistance against pathogens and parasites. Several mechanisms have been proposed to explain the maintenance of high allelic diversity in MHC genes(3-5). MHC-based sexual selection strategies(6) are known to involve olfactory mechanisms in fish(7,8), mice(9), and possibly humans(10). Using male odours to guide their mate choice, females of the three-spined stickleback (Gasterosteus aculeatus) optimize the degree of MHC poly-morphism in their off-spring(7,8); however, the molecular nature of odour signals is unknown.

Body odour and reproductive behaviour are influenced by the highly polymorphic genes of the major histocompatibility complex (MHC) in many species(11,12). MHC molecules sample, for presentation at the cell surface, a certain subset of intracellular peptides; the chemical diversity of bound peptide populations is directly determined by the molecular structure of ligand binding grooves of MHC molecules(2). Therefore, information for individual recognition may be conferred either by the polymorphic MHC molecules themselves or by the chemical properties of their ligands. Whatever their molecular nature, in an experimental situation, odorant(s) that function in MHC-related sexual selection can be expected to have at least two properties. First, they should be able to modify the outcome of mate choice. Second, this effect should depend on the MHC status of mating partners. While MHC-related mating preferences for mice(9) and humans(10) are known for some time, this phenomenon was only recently described in fish(7,8). We have used this experimental system and the above criteria to identify potential odour signals.

We show that mate choice can be experimentally modified by synthetic peptides resembling MHC ligands. In most cases, gravid female sticklebacks preferred water of males that was supplemented with peptides to un-supplemented male water. However, when the addition of peptides to male water led to the rejection by the female, the combined MHC diversity (as estimated by class-IIB alleles) was significantly higher than in pairs preferring the peptide supplemented water. Thus, peptides function as odour signals for female sticklebacks when they assess the degree of MHC polymorphism of their potential mate.

The result that MHC ligands, particularly peptidic MHC ligands are capable of modifying bodily odours is completely surprising. So far peptides have not been taken into account as possible odorous substances due to their mass. Instead of this, it was assumed that the commensurate microorganism flora of an individual can be influenced by the MHC structure and thus via the microorganism MHC-specific low-molecular metabolic products are produced, and the latter represent part of the bodily odour(10,17,18).

Thus, the subject matter of the present invention is the use of MHC ligands for the manufacture of an agent for modifying bodily odours. The invention is suitable for modifying bodily odours in animals, particularly in vertebrates such as fish, amphibia, reptiles, birds and mammals including human. The modification of bodily odours may result in modification of mate choices.

The MHC ligand may be an MHC class I or MHC class II ligand. Preferably, the MHC is an MHC class II ligand. The MHC ligand may be a peptide, particularly a synthetic peptide. The peptide has preferably a length of from 5-20 amino acids, more preferably from 6-15 amino acids. Further, the peptide preferably exhibits anchor-amino acid residues required for the binding to allele-specific MHC molecules(2). Further, the MHC ligand may be a peptide derivative, i.e. a substance which has peptide-like MHC binding characteristics, but which has been chemically modified. Methods of producing peptide derivatives which may act as MHC ligands are described in reference(2) and citations therein. Furthermore, the MHC ligands may be synthetic non-peptidic compounds capable of binding to MHC molecules.

A preferred embodiment of the invention relates to the fields of cosmetics, particularly the manufacture of a perfume. Up to now the composing of perfumes was performed with natural or synthetic basic materials, based on empirical knowledge. In the light of the findings underlying the present invention the bodily odour can be influenced in a predetermined manner by means of adding defined MHC ligands or rather combinations of several MHC ligands to perfumes. Moreover, synthetic peptides can completely or at least partially substitute the costly substances present in perfumes so far, in order to achieve a cheaper and chemically defined composition of perfumes.

Cosmetic compositions according to the invention contain common cosmetic components, such as further active substances, carriers, diluents and/or adjuvants, apart from the MHC ligands. For example the cosmetic compositions may be formulated as liquids, gels, creams, slurries, ointments, aerosols, sprays, suspensions, powders etc. together with other ingredients, e.g. further odorants, auxiliaries, carriers and/or diluents. The compositions may be administered topically, orally or nasally or by any other suitable means. Preferably, the compositions are applied to skin and/or clothes of an individual in a manner that allows access of the compound to the environment, e.g. air space, enclosing the individual. The concentration of the active substance in the cosmetic compositions preferably is preferably in the subnano to micromolar range and more preferably within the range of from 0.01 nM to 100 µM.

In a further embodiment of the invention compositions for modifying bodily odours, which comprise MHC ligands as an active agent, may be used for therapeutic applications in human and veterinary medicine, e.g. for use in reproductive medicine. For example, it is known that couples having a high number of early abortions exhibit a great identity of their MHC types (reference 12 and citations therein). Thus, when employing different synthetic MHC peptides for one partner, a reduction of the rate of abortions may be achieved. Moreover, further applications in human medicine and veterinary medicine are conceivable, such as the increase of mating success in selected breeding.

The compositions for therapeutic applications contain the active substance in concentrations which are preferably in the subnano- to micromolar range and more preferably within the range of 0.01 nM to 100 µM. Apart from this, the compositions can also contain pharmacologically acceptable carriers, diluents and/or adjuvants. The therapeutic compositions may be administered for instance by any means that allows access of the compound to the environment, e.g. air space, enclosing the individual, e.g. as described above for cosmetic compositions.

Furthermore, the invention shall be illustrated in detail by the following Figures and Examples.

### Figure 1

Peptides are recognized as odour cues in mate choice. a, Peptides with structural features of MHC ligands modify mate choice. Gravid female sticklebacks were tested with male water with (left panels) and without (right panels) addition of synthetic peptides. The excess time (in seconds) spent in each category is shown (0 indicates that the fish spent 300 seconds each in the two columns of the flow chamber). Five peptides (FAPGNYPAL, ASNENMETM, AAPDNRETF, SYFPEITHI and SYIPSAEKI) were added to a final concentration of 5nM. Overall, females (n = 24) spent a mean of 368.9+/-36.5 sec. in the column containing the peptide mixture. b, MHC diversity of mating pairs determines the outcome of mate choice after peptide supplementation. The total number of different MHC class-IIB alleles in pairs of female and male fish was compared for groups of females preferring or rejecting peptide supplemented male water. MHC diversity is higher in pairs where the female rejected the peptide supplemented water. The box plots indicate median, quartiles and deciles.

### Table 1

Natural male odours and synthetic peptides display similar characteristics in mate choice experiments.

### EXAMPLE

### 1. Methods

### 1.1 MHC characterization

The characterization of MHC class-IIB genes was performed as described(7,29).

### 1.2 Sexual selection experiments

The source of sticklebacks has been described(7). The flow channel set-up to evaluate mate choice determines odour preference, as a precursor of mate choice. Water was taken from the tank of a single male per trial and used both as stimulus (after addition of peptide(s)) .and control (after addition of solvent) water. Peptides were added to the bottle with stimulus water just before the experiment at a final concentration of 1mM; this becomes diluted to 5nM in the water column passing through the test compartment. The following nona-peptides were used, MHC specificities indicated in brackets: FAPGNYPAL (H2-K^{b}); ASNENMETM and AAPDNRETF (H2-D^{b}); SYFPEITHI and SYIPSAEKI (H2-K^{d}). Peptides were synthesized by solid-phase 9-fluorenylmethoxycarbonyl chemistry. In experiments using a single peptide, AAPDNRETF was used. All experiments were performed in double-blinded fashion as described. Statistical analysis was performed with JMP 4.

### 2. Results

The binding preferences for peptides of MHC molecules of sticklebacks are unknown; therefore synthetic peptides derived from endogenous mouse and viral proteins that are ligands for different mouse MHC molecules were used. Such peptides almost certainly appear foreign to sticklebacks irrespective of their MHC genotypes. Gravid females prefer water from a tank harbouring a male to that from a tank with fresh water (Experiment A, Table 1). Therefore, it was tested whether addition of peptide(s) to water from individual males would alter the outcome of mate choice. Gravid females were presented with water from single males with and without the addition of a mixture of five different peptides in the two columns of a flow-channel.

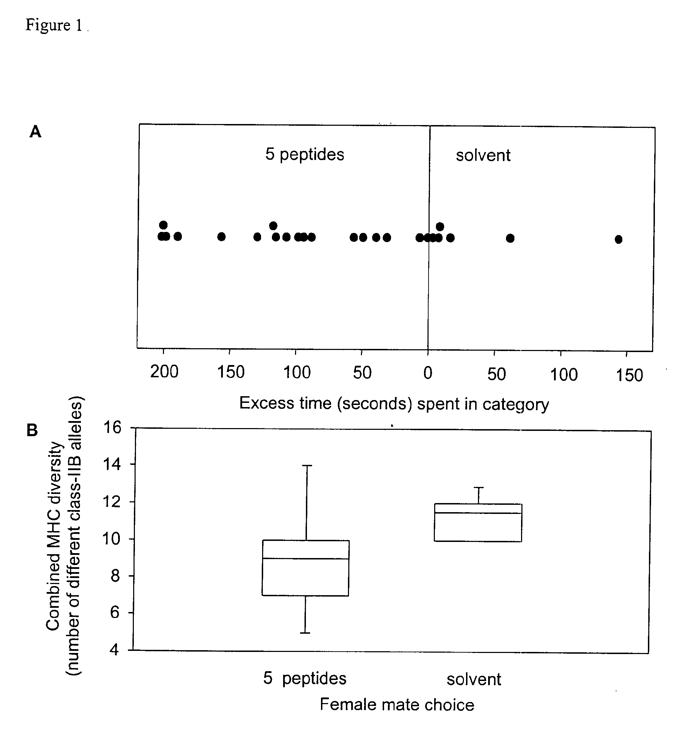

Most females preferred the spiked male water to its mock-supplemented version (Fig. 1a), indicating that synthetic peptides are capable of modifying mate choice. Overall, the preference for peptide-supplemented male water was highly significant (368.9+/-36.5 sec. vs. 231.1 +/-36.5 sec.; matched-pair t-test: t=3.8, n=24, p=0.001, two-tailed) (Table 1). However, in certain combinations of females and males, the female rejected the peptide-supplemented male water (Fig. 1a). Because female sticklebacks guide their mate choice towards an optimal degree of MHC polymorphism in their offspring(7,8), the experimental intervention would distort the preferred degree of MHC diversity during mate choice, if females accepted synthetic peptides as odour signals. The known genotypes at MHC class-IIB loci(7,13) were used in order to test this hypothesis. The results shown in Fig. 1b indicate that the combined MHC polymorphism represented in the pairs preferring plain male water is significantly higher than in the pairs preferring the water samples supplemented with peptides (11.5 (10, 12.25) different alleles vs. 9 (7, 10) different alleles; median (quartiles); Wilcoxon matched pair exact test: z=1.96, n=21, p = 0.046, two-tailed). It should be noted that mating pairs in the group rejecting the supplemented water would, on average, pass on to their offspring a number of detectably different class-IIB alleles closer to the average number observed in the population of wild-caught fish (5.8+/-0.13; Ref. 7) than pairs from the group preferring the peptide supplement. The latter are thus attracted by the peptide supplemented water as this mimics increased MHC diversity in the male. This shows interaction of synthetic peptides and natural odour signals. It is also further evidence for the optimization strategy(7,8) used by sticklebacks during mate choice, the optimal number of different MHC class-IIB alleles being between 9 and 11.5.

When the above experiment was repeated with one peptide (Experiment C, Table 1), female sticklebacks again preferred the supplemented male water (327.7+/-24.8 sec. vs. 272.3+/-24.8 sec.; matched-pair t-test: t=2.2, n=25, p=0.035, two-tailed). Importantly, there is a strong trend that the excess time spent on the side of single peptide-supplemented male water is lower than that with five peptides (27.7+/-12.4 sec. vs. 68.9+/-18.2 sec.; matched-pair t-test: t=1.9, n=49, p=0.066, two-tailed). Given a similar distribution of MHC diversity in mating pairs, this is to be expected, because a single peptide only marginally increases the presumptive odour complexity.

The properties of peptide signals perceived by female sticklebacks in odour-related mate choice were further investigated in the following experiments. First, the effect of peptides in plain tank water was determined. The results show that the addition of peptides to plain tank water is not attractive to female sticklebacks (Experiment B, Table 1). Rather, peptide(s) are only recognized in conjunction with male water (Experiments C and D, Table 1), indicating the presence of other, possibly invariant male odour signals. When non-gravid fish were exposed to male water with and without peptide, no preference was observed (Experiment E, Table 1), suggesting that gravidity is an important parameter to activate the recognition system. Importantly, experiments B and E (Table 1) also rule out the possibility that females recognize peptides out of the context of mate choice, i.e. as food signals etc.

Both the allele counting(7) and the dis-assortative mating(8) strategies used by sticklebacks require the assessment of the diversity of MHC peptide ligands. Although it is unknown at present whether peptides can be recognized by the olfactory system, a special class of olfactory receptors (termed V2R in mice) structurally are candidates for binding small peptides(14). Indeed, preliminary evidence indicates that receptors of the V2R class in mice interact with pheromonal components of undefined proteinaceous nature(15). For one V2R homologue in fish, high affinity binding to the amino acid arginine was found(16), but the effect of peptides or proteins was not investigated in this experiment. Due to their vast chemical diversity, peptides are much more likely to carry individual-specific information than the small compounds (for instance metabolites of amino acids) that have pre-viously been considered as signal molecules(17,18). Olfactory receptors are exquisitely sensitive, and recognize their ligands in the sub-nanomolar concentration range(14). Although thousands of chemically different peptides are presented by a particular MHC molecule on the cell surface(19), some peptides are much more frequent than others(20) and may be present in concentrations amenable to olfactory detection. For identification of MHC diversity, olfactory discrimination of such prevalent peptides may suffice.

MHC-bound peptides may become ligands for olfactory receptors in at least two ways. They may be released from MHC molecules and then bind to non-specific carrier proteins(21); in such a way, they may survive enzymatic degradation during transit in extracellular fluids and bodily secretions(22,23). In addition to the polymorphic MHC molecules as primary determinators of peptide diversity, polymorphic carrier proteins such as MUPs(24) may further modify the ligand repertoire(25,26) presented to olfactory receptors. An alternative but non-exclusive possibility is that peptides may remain bound to soluble MHC molecules or fragments thereof that have been detected in serum(27) and urine(28). Irrespective of the molecular nature of the carrier molecule(s), the recognition of carrier-bound peptides by olfactory receptors may occur in ways analogous to recognition by T-cell receptors.

In conclusion, the results suggest that peptides with structural features of MHC ligands constitute an individual-specific part of male odour in sticklebacks and also provide a conceptual frame-work to explain the results of previous experiments on MHC-related reproductive behaviour in other species, such as rodents and human.

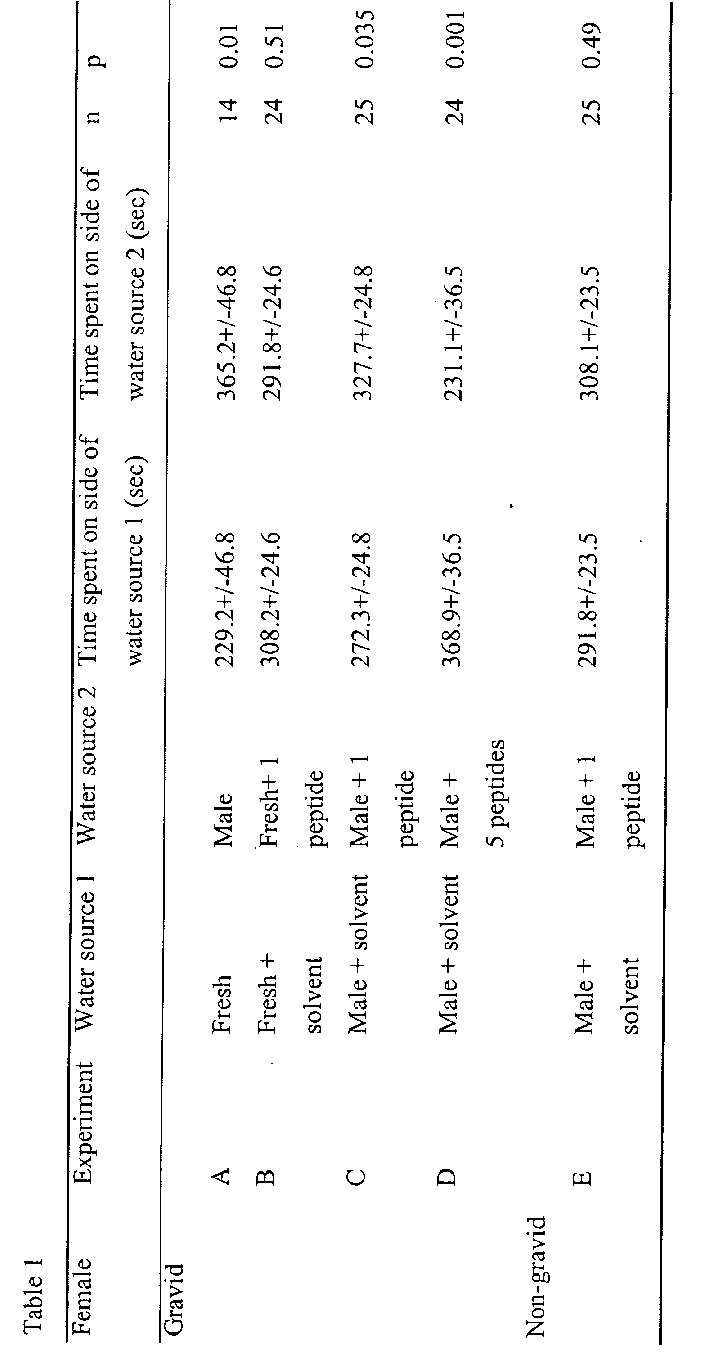

### REFERENCES

- 1.: Little & Parham. Rev. Immunogenet. 1, 105-123 (1999).
- 2.: Rammensee et al. MHC ligands and peptide motifs. (Landes Bioscience, Georgetown, 1997).
- 3.: Apanius et al. Crit. Rev. Immunol. 17, 179-224 (1997).
- 4.: Klein. Natural History of the Major Histocompatibility Complex (Wiley, New York, 1986).
- 5.: Edwards & Hedrick. Trends Ecol. Evol. 13, 305-311 (1998).
- 6.: Penn & Potts. Am. Nat. 153, 145-164 (1999).
- 7.: Reusch et al. Nature 414, 300-302 (2001).
- 8.: Aeschlimann et al., submitted for publication.
- 9.: Yamazaki et al. J. Exp. Med. 144, 1324-1335 (1976).
- 10.: Wedekind & Furi. Proc. R. Soc. London B. Biol. Sci. 264, 1471-1479 (1997).
- 11.: Penn & Potts. Adv. Immunol. 69, 411-436 (1998).
- 12.: Singh. Reproduction 121, 529-539 (2001).
- 13.: Sato et al. Gastrosteus aculeatus. Mol. Mar. Biol. Biotechnol. 7, 221-231 (1998).
- 14.: Firestein. Nature 413, 211-218 (2001).
- 15.: Krieger et al. J. Biol. Chem. 274, 4655-4662 (1999).
- 16.: Speca et al. Neuron 23, 487-498 (1999).
- 17.: Singer et al. Proc. Natl. Acad. Sci. USA 94, 2210-2214 (1997).
- 18.: Montag et al. Proc. Natl. Acad. Sci. USA 98, 9249-9254 (2001).
- 19.: Bevan. Immunity 7, 175-178 (1997).
- 20.: Yewdell & Bennink. Ann. Rev. Immunol. 17, 1-30 (1999).
- 21.: Robertson et al. J. Chem. Ecol. 19, 1405-1416 (1993).
- 22.: Hayakawa et al. J. Hered. 74, 453-456 (1983).
- 23.: Utsumi et al. J. Neurobiol. 39, 227-236 (1999).
- 24.: Robertson et al. Biochem. J. 316, 265-272 (1996).
- 25.: Hurst et al. Nature 414, 631-634 (2001).
- 26.: Brennan et al. Neuroscience 90, 1463-1470 (1999).
- 27.: Wobst et al. Genetica 104, 275-283 (1998).
- 28.: Singh et al. Nature 327, 161-164 (1987).
- 29.: Binz et al. J. Fish Biol. 58, 887-890 (2001).

## Claims

1. Use of MHC ligands for the manufacture of an agent for modifying bodily odours.

2. The use of claim 1 for the manufacture of a cosmetic composition.

3. The use of claim 2 wherein the cosmetic composition is a perfume.

4. The use of claim 1 for the manufacture of a therapeutic composition.

5. The use of claim 4 wherein the therapeutic composition is a composition for use in reproductive medicine.

6. The use of claim 4 or 5 for use in human or veterinary medicine.

7. The use of any one of claims 1-6 wherein the MHC ligand is an MHC class I or an MHC class II ligand.

8. The use of any one of claims 1-7 wherein the MHC ligand is a peptide or a peptide derivative or a synthetic non-peptidic compound capable of binding to MHC molecules.

9. A method for modifying bodily odours comprising applying a composition comprising at least one MHC ligand as an active agent to a subject in need of modifying bodily odours.

10. The method of claim 9 comprising application by topical means.

11. The method of claim 9 comprising application as an aerosol.

12. A composition for modifying bodily odours comprising at least one MHC ligand as an active agent.

13. The composition of claim 12 which is a cosmetic composition.

14. The composition of claim 12 which is a therapeutic composition.
